# EUROPEAN PATENT APPLICATION

(11) **EP 2 815 770 A1**
(43) Date of publication of application: **24.12.2014**
(21) Application number: 14169448.9
(22) Date of filing: 22.05.2014
(51) Int. Cl.: A61L 9/04, A61L 9/12, B65D 83/14, B65D 47/42, A45D 34/00

(54) **Container for deodorants**

(30) Priority: 24.05.2013 IT MI20130851
(71) Applicant: RE.LE.VI. S.P.A., 46040 Rodigo (Mantova) (IT)
(72) Inventor: Venini, Roberto Cherubino, 20121 Milano (IT)
(74) Representative: Petraz, Gilberto Luigi

(57) **Abstract**

A container for deodorants comprises a front portion (11) and a rear portion (13) defining a zone (22) for housing deodorant able to spread through the air by means of the passage of air through through holes (12) present at least in said rear portion (13). At least in one of its other zones, the container (10) has at least one collection compartment (19).

## Description

### FIELD OF THE INVENTION

The present invention concerns a container for deodorants, specifically deodorants for automobiles, contained in suitable replaceable cartridges, transpirant and with a liquid content, which are affixed in proximity to the air vents so that they spread fragrances into the environment due to the passage of air through through holes present in at least part of said container.

In particular, the container for deodorants according to the present invention, in at least one zone, has a shape specifically designed to define a compartment, able to collect accidental spillage of the liquid content of said cartridges, possibly all such spillage, and also excess condensation of said liquid, leakages or similar losses of liquid which can occur during use.

### BACKGROUND OF THE INVENTION

Multiple types of containers for deodorants for automobiles are known in the state of the art. Some of these contain solid deodorants, others semi-solid ones, such as gels or suchlike, still others contain liquid, generally associated to a transpirant membrane. In some cases, the containers are replaced when the deodorant contained therein finishes, in other cases the containers can be re-used since the deodorant which has been consumed can itself be replaced or because it is contained in replaceable cartridges.

All these containers are generally positioned in proximity to the air vents of the automobile, with known systems, inasmuch as part of the air which is introduced inside the automobile passes - through suitable through holes - through the deodorant container, thus spreading the deodorant fragrance until it has all been consumed.

In this known process, at least part of the deodorant in the liquid and substantially liquid state does not achieve the correct aerial diffusion, and it often happens that part of the liquid deodorant not spread leaks outside its container.

It can also happen that, in the assembly step, during usage, or when being removed, the transpirant membrane partly or totally breaks, thus causing an accidental leakage of the liquid deodorant, even all of it.

The leaking deodorant then dirties both its container and also the furnishings in the automobile below it and near it, and also - if it is not removed quickly and correctly on the basis of its composition - can ruin the furnishings of the automobile thus affected.

As we mentioned, the damage can be greater either because of the corrosiveness of the deodorant or because of how the leaked deodorant is removed.

To this it must be added that the esthetic quality is negatively affected and there is a sense of dirtiness: these perceptions are exactly the opposite of what such types of diffusers are intended to give.

As we said, the unwanted presence of liquid fragrance outside its container can also occur due to accidental lacerations of the container, or due to any other erroneous form of assembly or use of the device; it can also be caused by condensation outside the container, connected to differences in temperature that can occur in the interior of the automobile.

Document US-A-6,254,836 describes an air freshener device and fragrance dispenser which can be hung in an automobile. This device comprises a circular bulb on the back and contains an absorbent material housed in a recess and impregnated with an oily fragrance, an air chamber being provided delimited by a wall with holes for the passage of the air with the fragrance. However, this known solution has all the common defects of the state of the art, since an accidental leakage or accumulation of deodorant can easily come out, for example through said holed wall, since there is nothing provided to stop the liquid from coming out.

Document GB-A-2.371.749 describes an apparatus to disperse an air modifier agent, for example an air freshener, insecticide or insect repellent, which uses a super-absorbent polymer as the reserve of said agent. In particular, a reserve cushion of this agent is provided housed in a container with a portion shaped like a cup, a front part sealed to the cup shaped portion and provided with apertures. This solution provides to pour water on the reserve cushion to dampen it and allow the spread of the agent. Therefore this known solution also entails the risk of liquid leaking out.

Document US-A-2011/0057053 describes a perfume diffuser associated to a mobile phone or to a portable camera, which comprises a housing to receive a tablet or cartridge impregnated with a perfume or deodorant and associated to a closing lid. This known solution too is not without the risk of spills of liquids or condensation.

One purpose of the present invention is to prevent the deodorant located inside its container from coming out therefrom in any way other than desired.

Another purpose of the present invention is to prevent the deodorant which has leaked in this way from affecting the external part of its container, thus ruining it, so that it cannot then be re-used.

Another purpose of the present invention is to prevent the deodorant, as it comes out, from dripping or in any case falling on part of the automobile, for example the dashboard, the gears unit, the installed or auxiliary electronic apparatuses etc., thus ruining it through corrosion and/or because it is removed with the wrong products.

Another purpose is to prevent the deodorant, coming out from its container in an unwanted way, from generating a sense of dirtiness and neglect.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In order to obtain the above purposes, the container for deodorants according to the present invention, in at least one of its zones, assumes a shape able to accommodate the deodorant which would otherwise leak from the container.

The shape of the container defines a compartment to collect the liquid arriving accidentally from the deodorant, or condensation, in which, a fraction or all the liquid deodorant can be collected which, for the reasons stated above or due to any other anomalous situation, would otherwise come out improperly, or where possible condensation can accumulate. The collection compartment can be provided in a lower zone, during use, of the container and/or in proximity to through holes for the air to come out.

The collection compartment can be provided in the front and/or rear portion of the container, or can be obtained by the cooperation of two parts, even reciprocally mobile, of the front and/or rear portion.

The collection compartment can have any shape suitable for its function and can be associated to lead-ins, for example channels, partitions, ridges or suchlike, always present on the container, able to convey into the collection compartment condensation and/or deodorant coming out from the container which would not be spread through the air.

In one formulation of the invention, the collection compartment has a capacity such as to contain the whole amount of liquid deodorant provided for that specific container. According to a variant, the collection compartment is suitable to house a suitable absorbent material covering at least its bottom and able to increase the containing capacity of the compartments. The absorbent material can be attached or replaceable during the lifespan of the container.

In a variant, the bottom of the collection compartment can be equipped with means for positioning and/or retaining the absorbent material.

The absorbent material can be any known material with this technical function; merely by way of example it can be made of fabric, paper, synthetic products, with a simple or complex structure such as, for example, a honeycomb structure.

In another variant, the collection compartment can also be provided with suitable holes able to allow the liquid deodorant contained to spread through the air, once the cartridge of deodorant has been consumed, or constantly, when optimum diffusion conditions are achieved.

In one formulation, the invention is applied for deodorants of the liquid type, contained inside a transpirant membrane, of the osmotic type for example, or the absorbent type or other similar or comparable type to these.

These and other aspects, characteristics and advantages of the present disclosure will be better understood with reference to the following description, to the drawings and to the attached claims. The drawings which are integrated with and are part of the present description show some forms of embodiment of the present invention and, together with the description, are given in order to describe the principles of the disclosure.

The various aspects and characteristics described in the present description can be applied individually, where possible. These individual aspects, for example aspects and characteristics described in the attached dependent claims, can also be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some forms of embodiment, given as a non-restrictive example with reference to the attached drawings wherein:
- fig. 1 is a front view of forms of embodiment of a closed container according to the present description;
- fig. 2 is a side view of forms of embodiment of a closed container according to the present description;
- fig. 3 is a rear view of forms of embodiment of an open container according to the present description;
- fig. 4 is a side view of forms of embodiment of an open container according to the present description;
- fig. 5 is a rear perspective view of forms of embodiment of an open container according to the present description;
- fig. 6 is a section of forms of embodiment of an open container according to the present description;
- fig. 7 is a rear perspective view of forms of embodiment of a closed container according to the present description;
- fig. 8 is a section of forms of embodiment of a closed container according to the present description;
- fig. 9 is a front perspective view of forms of embodiment of a closed container according to the present description;
- fig. 10 is a section of forms of embodiment of a closed container according to the present description in the event that liquid is collected therein.

To facilitate comprehension, the same reference numbers have been used, where possible, to identify identical common elements in the drawings.

It is understood that elements and characteristics of one form of embodiment can conveniently be incorporated into other forms of embodiment without further clarifications.

### DETAILED DESCRIPTION OF SOME FORMS OF EMBODIMENT

We shall now refer in detail to the various forms of embodiment of the present invention, of which one or more examples are shown in the attached drawing. Each example is supplied by way of illustration of the invention and shall not be understood as a limitation thereof. For example, the characteristics shown or described insomuch as they are part of one form of embodiment can be adopted on, or in association with, other forms of embodiment to produce another form of embodiment. It is understood that the present invention shall include all such modifications and variants.

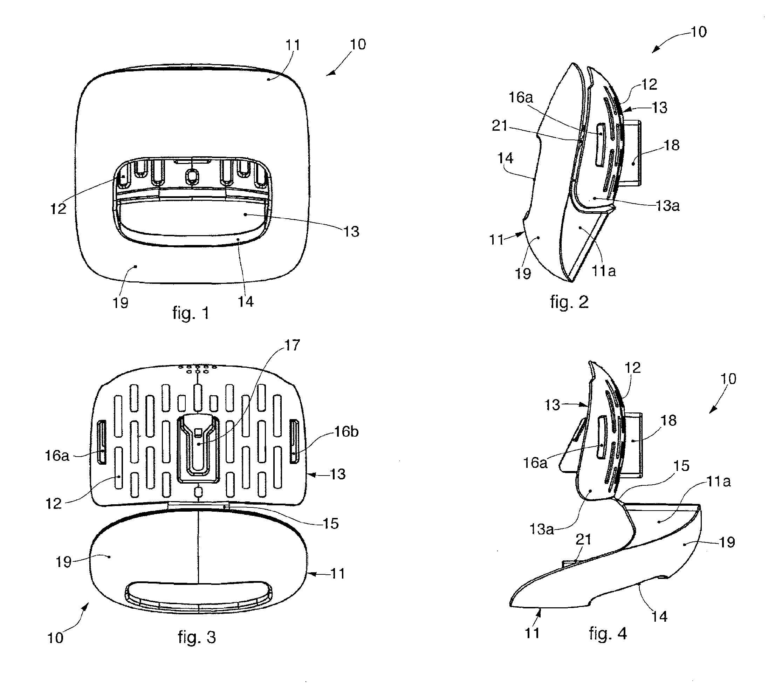

Fig. 1 is used to describe forms of embodiment of a container 10 for deodorants according to the present description, which has a deodorant inside with its own case, or a cartridge containing the deodorant, for example having a transpirant membrane, in particular in a zone suitable to contain said deodorant. The deodorant is able to spread through the air by means of a stream of air, preferably a stream of forced air, as for example in the case of air vents for example of an automobile. In possible implementations, said deodorant can be liquid.

The container 10 includes a front portion 11 in which, in the specific case shown by way of example, there is for example a through hole 14, present for example on the front side of the container 10. A possible function of the through hole 14 is to render the deodorant visible during use, with its casing, a transpirant membrane for example, disposed inside the container 10.

The possibility of having several through holes on the front side is an alternative solution, which can be provided in a regular manner, for example in rows or columns, or according to other desired geometries, in a decorative pattern or simply random.

According to forms of embodiment described here, the container 10 also includes a rear portion 13.

In some forms of embodiment the front portion 11 and the rear portion 13 define a zone 22 to house the deodorant.

With reference for example to fig. 1, the rear portion 13 of the container 10 is visible through the through hole 14. According to possible implementations, the rear portion 13 can be, in this case, selectively mobile with respect to the front portion 11. According to possible implementations, in the rear portion 13, by way of a non-restrictive example regarding the number, type or position of hole, rear through holes 12 are provided which allow the deodorant to spread into the environment.

Fig. 2 is used to describe forms of embodiment in which, by way of non-restrictive example, the container 10 has a closed shell shape, able to determine, between the front portion 11 and the rear portion 13, said zone 22 suitable to contain the deodorant, or the cartridge containing the deodorant, for example having a transpirant membrane.

In some forms of embodiment, there can also be a spacer 18 in the rear portion 13 which - cooperating with an air vent of the automobile where the container 10 can for example be housed - allows the same container 10 to have a position of use lying on a transverse plane, for example substantially perpendicular, to the plane of the automobile.

According to possible implementations, the rear portion 13 can include a holed part 13a which during use, that is, when the front portion 11 and the rear portion 13 are closed, aligns to a non-holed part 11a of the front portion 11 (fig. 2).

Fig. 3 is used to describe forms of embodiment in which the container 10 is in an open position. With reference to fig. 3, an elastic joint 15 is provided that hinges, articulates or rotatably connects the front portion 11 to the rear portion 13. The elastic joint 15 allows to move the front portion 11 with respect to the rear portion 13. In order to take the container 10 into a closed position and stably maintain it in this position, the rear portion 13 can have closing holes 16a and 16b which cooperate with mating clamping fins 21 provided in a corresponding position in the front portion 11.

With reference to fig. 3, the through holes 12 are once again provided in the rear portion 13, and attachment means 17 can also be provided, such as a clip for example, able to removably fasten the container 10 to a support, for example to the air vents of the automobile, not shown here. In this way, the container 10 can be installed suspended or hanging.

Figs. 4, 5, 6, 7, 8 and 9 are used to describe forms of embodiment combinable with all the forms of embodiment described here, in which the container 10 is provided, in its part that is lower during use, with a compartment 19 to collect the liquid coming accidentally from said deodorant, or also condensation, for example of the liquid deodorant which may have accidentally come out, and/or of the condensation that has formed following particular thermal or climatic conditions, created for example inside the automobile.

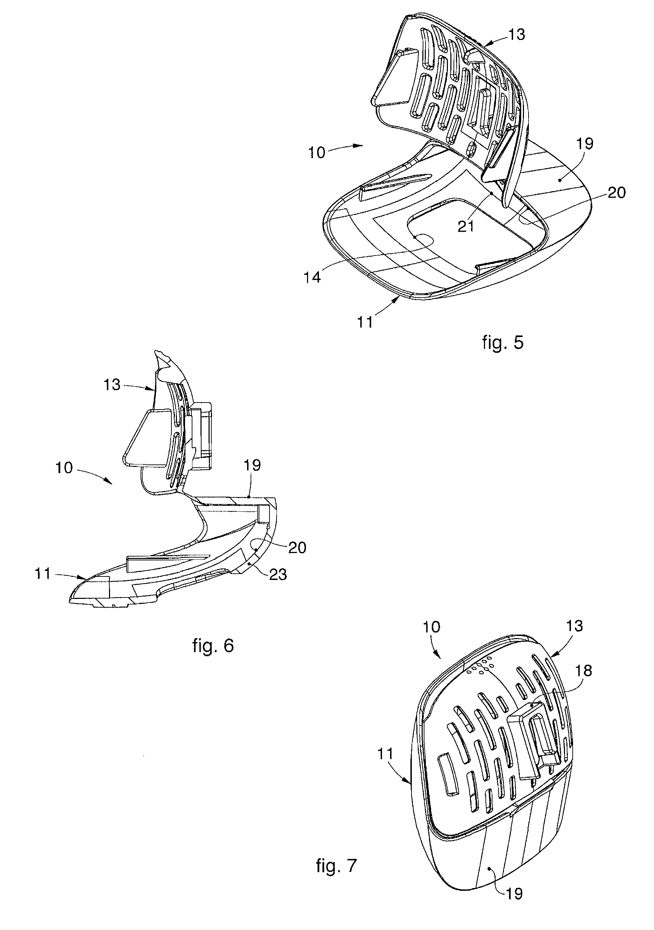

The collection compartment 19 includes, in forms of embodiment described here with reference to the attached drawings, an internal chamber 20 to receive the liquid, delimited by a wall 23, in particular a shaped wall, advantageously without holes, so as to prevent any leakage of the liquid (see for example figs. 5, 6 and 8). The wall 23, advantageously without holes, that delimits the chamber 20 can have, for example, a rounded shape in a lower part, closed and without holes, of the front portion 11 of the container 10.

The collection compartment 19 is preferably sized so as to be able to house at least the entire quantity of liquid deodorant present inside the specific container 10 on which the collection compartment 19 is made.

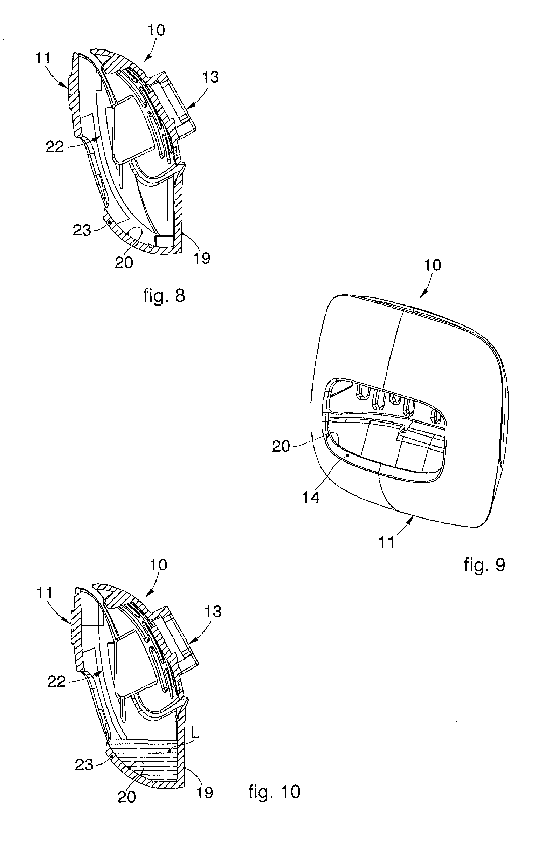

For example, in fig. 10, the case is clearly shown where liquid L accumulates or collects in the collection compartment 19, thus filling part of the chamber 20, which keeps the liquid L confined, therefore preventing it from leaking in an unwanted manner from the container 10.

With reference for example to fig. 4, forms of embodiment are also described in which the container 10, at least partly, can have at least a holed part 13a of the rear portion 13 at least partly removable from a first closed position (figs. 1, 2, 7, 8 and 9) and a second open position (figs. 3, 4, 5 and 6).

Between the removable holed part 13a of the rear portion 13 and the non-holed part 11a of the front portion 11 the cited elastic joint 15 can be provided.

The open position of the rear portion 13 can for example facilitate both the cleaning of the container 10 and also the possibility of replacing the deodorant and/or the absorbent material, not shown here.

In some forms of embodiment, the front portion 11 and the rear portion 13 are connected in an articulated way by means of said elastic joint 15, so as to be reciprocally mobile between the closed position and the open position. In particular, the front portion 11 and the rear portion 13 can define said zone 22 in the closed position.

In forms of embodiment described using the attached drawings, the rear portion 13 can be provided with the attachment means 17 in order to removably connect the container 10 to a support wall, in particular when the front portion 11 and the rear portion 13 are in the closed position. Advantageously, the attachment means 17 are configured to achieve a connection to the support wall in such a way that, in the closed position, the collection compartment 19 is disposed completely under the zone 22 to house the deodorant.

It is clear that modifications and/or additions of parts may be made to the container 10 as described heretofore, without departing from the field and scope of the present invention.

For example, the collection compartment 19 can be made, depending on the different shapes of the container 10, wholly in a non-holed part of the rear portion 13, or partly in the rear portion 13 and partly in the front portion 11.

It is also clear that, although the present invention has been described with reference to some specific examples, a person of skill in the art shall certainly be able to achieve many other equivalent forms of container 10, having the characteristics as set forth in the claims and hence all coming within the field of protection defined thereby.

## Claims

1. Container for deodorants, comprising a front portion (11) and a rear portion (13) defining a zone (22) for housing deodorant able to spread through the air by means of the passage of air through through holes (12) present at least in said rear portion (13), **characterized in that**, at least in one of its other zones, said container (10) has at least one compartment (19) to collect liquid coming accidentally from said deodorant, or condensation.

2. Container as in claim 1, **characterized in that** said collection compartment (19) is provided in the front portion (11) of said container (10).

3. Container as in claim 1, **characterized in that** said collection compartment (19) is provided in the rear portion (13) of said container (10).

4. Container as in claim 1, **characterized in that** said collection compartment (19) is obtained by the union of at least one part of the front portion (11) and of at least one unperforated part of the rear portion (13) of said container (10).

5. Container as in any claim hereinbefore, **characterized in that** said collection compartment (19) has sizes such as to be able to contain the whole deodorant contained inside the container (10).

6. Container as in any claim hereinbefore, **characterized in that** said collection compartment (19) cooperates with lead-ins able to promote the collection of the deodorant and/or condensation.

7. Container as in any claim hereinbefore, **characterized in that** said collection compartment (19) cooperates with absorbent material.

8. Container as in claim 7, **characterized in that** the absorbent material is located inside the collection compartment (19).

9. Container as in claim 7 or 8, **characterized in that** the absorbent material can be replaced during the use of the container (10).

10. Container as in any claim from 7 to 9, **characterized in that**, on the bottom, the collection compartment (19) has means for positioning and/or retaining the absorbent material.

11. Container as in any claim hereinbefore, **characterized in that** the front portion (11) and the rear portion (13) are connected in an articulated manner by means of an elastic joint (15), so as to be reciprocally mobile between a closed position and an open position.

12. Container as in claim 11, **characterized in that** said rear portion (13) is provided with attachment means (17) to connect said container removably to a support wall when the front portion (11) and the rear portion (13) are in the closed position.

13. Container as in claims 11 and 12, **characterized in that** said attachment means (17) are configured to achieve a connection to said support wall so that, in said closed position, said collection compartment (19) is disposed completely under said deodorant housing zone (22).

14. Container as in any claim hereinbefore, **characterized in that** said collection compartment (19) comprises a chamber (20) delimited by a wall (23).

15. Container as in claim 11, **characterized in that** said wall (23) is without holes.

16. Container as in any claim hereinbefore, **characterized in that** said deodorant is liquid.
